# EUROPEAN PATENT APPLICATION

(11) **EP 4 365 275 A1**
(43) Date of publication of application: **08.05.2024**
(21) Application number: 22832493.5
(22) Date of filing: 22.03.2022
(51) Int. Cl.: C12M 1/00, C12M 3/00, C12N 5/079, C12Q 1/02

(54) **MEDICINAL AGENT EFFECT PREDICTION SYSTEM**

(30) Priority: 29.06.2021 JP 2021107213
(71) Applicant: Tohoku Institute of Technology, Sendai-shi, Miyagi 982-8577 (JP); Ushio Denki Kabushiki Kaisha, Tokyo 100-8150 (JP)
(72) Inventor: SUZUKI,Ikuro, Sendai-shi, Miyagi 982-8577 (JP); YAMANAKA,Makoto, Tokyo 100-8150 (JP)
(74) Representative: Maiwald GmbH
(86) International application number: PCT/JP2022/013098
(87) International publication number: WO 2023/276330

(57) **Abstract**

Provided is a system capable of easily and efficiently predicting the effect of a medicinal agent on nerves. The medicinal agent effect prediction system according to the present invention includes: a microfluidic device having a first culture space capable of culturing nerve cells and a second culture space capable of culturing neurites separately from the nerve cells; a storage unit that records a learned model based on a plurality of teaching data obtained by associating input data for learning, which is based on images of neurites in the second culture spaces respectively growing from nerve cells cultured in a state where each of known substances is individually added to a culture solution, with output data for learning which relates to information about impaired nerve sites resulting from the known substances; and an assessment unit that outputs, by culturing nerve cells in a state where a test substance is added to a culture solution and applying input data for assessment based on a microscope image of neurites in the second culture space to the learned model, information derived from probabilities of occurrence of impairments of different nerve sites.

## Description

### TECHNICAL FIELD

The present invention relates to a system for predicting the effect of a medicinal agent and particularly relates to a system for predicting the effect of a medicinal agent on nerves such as peripheral nerves and central nerves.

### BACKGROUND ART

Drug discovery research includes a phase for assessing toxicity and safety as well as a phase for confirming the efficacy of a medicinal agent. An impairment of peripheral nerves due to the administration of a medicinal agent is undesirable because such an impairment has a great effect on the quality of life (QOL) of a patient. Therefore, it is important in drug discovery research to understand in advance effects on nerves such as peripheral nerves (neurotoxicity).

As a method for assessing neurotoxicity, there is a known method of forming neurites from nerve cells using a microfluidic device and observing drug stimulation and its response (see, for example, Patent Document 1 mentioned below). Patent Document 1 discloses a microfluidic device having a channel capable of culturing neurites (also called "axons") extending from cell bodies.

### PRIOR ART DOCUMENT

Patent Document 1: JP-B2-6430680

### SUMMARY OF THE INVENTION

### PROBLEMS TO BE SOLVED BY THE INVENTION

However, the channel disclosed in Patent Document 1 has a width of 100 µm to 150 µm and a height of 100 µm to 200 µm. Therefore, the width and height of the channel are larger than the thickness of neurites, and the width and height are close to each other. For this reason, when nerve cells are cultured in this channel, neurites assemble to form a bundle-like structure. When nerve cells are cultured in such a manner, each of the neurites growing from the nerve cells cannot individually be observed and analyzed.

Further, there is a conventionally known method in which nerve cells are cultured in a petri dish. Also, in this case, nerve cells and neurites overlap each other during culture. To culture nerve cells while avoiding such a phenomenon, the density of neurites in the petri dish needs to be reduced. However, such a state significantly differs from a living body due to a reduction in the function of cells. Further, variations occur depending on the states of individual nerve cells. For this reason, there is a concern about using the image of neurites cultured by this method for the assessment of the effect of a medicinal agent.

Another conceivable method is to culture nerve cells at high density to form a network structure of neurites. However, even in this case, nerve cells and neurites overlap each other, and therefore it is difficult to identify a site to be observed. Further, the manner of overlapping is irregular from test to test, and therefore it is difficult to perform a quantitative assessment based on an observation image obtained by this method.

Under the circumstances described above, a method for quantitatively assessing the effect of a medicinal agent on nerves such as peripheral nerves in vitro has not been established at this moment.

In light of the above problems, it is an object of the present invention to provide a system capable of easily and efficiently predicting the effect of a medicinal agent on nerves.

### MEANS FOR SOLVING THE PROBLEMS

The present invention is directed to a medicinal agent effect prediction system including:
a microfluidic device having a first culture space capable of culturing nerve cells and a second culture space that is provided so as to extend from the first culture space and that is capable of culturing, separately from the nerve cells, neurites growing from the nerve cells;
a storage unit that records a learned model generated by machine learning based on a plurality of teaching data obtained by associating input data for learning with output data for learning: the input data for learning being data which is based on microscope images of the neurites located in the second culture spaces and respectively growing from the nerve cells cultured in the first cu lture spaces of the microfluidic devices prepared for learning in a state where each of known substances that impair known nerve sites belonging to a group of impaired site candidates or do not impair any nerve site is individually added to a culture solution, the output data for learning being data which is information about impaired nerve sites resulting from the known substances added to the first culture spaces in which the nerve cells corresponding to the microscope images are cultured or information that there is no impaired site;
a data input receiving unit that receives input of input data for assessment based on a microscope image of the neurites located in the second culture space and growing from the nerve cells cultured in the first culture space of the microfluidic device prepared for assessment in a state where a test substance, which is not known as to which nerve site is impaired thereby, is added to a culture solution; and
an assessment unit that outputs information derived from probability of occurrence of impairment of each nerve site belonging to the group of impaired site candidates resulting from introduction of the test substance, by applying the input data for assessment to the learned model.

The microfluidic device is capable of culturing nerve cells in the first culture space and of culturing cell protrusions growing from the nerve cells in the second culture space. Therefore, the neurites do not form a bundle-like structure but are cultured in such a manner that the respective neurites are arranged in a width direction.

Therefore, a dense network structure of neurites can be observed from site to site by culturing nerve cells in this microfluidic device and particularly observing the second culture space.

When the microfluidic device is used to culture nerve cells in a state where a substance (known substance) that is known to have or not to have an effect on nerves or known to have an effect on a known nerve site is added, the effect of introduction of the known substance appears in the information of observation image (microscope image) of neurites present in the second culture space. Since the microfluidic device is capable of culturing neurites separately from nerve cells, the effect of introduction of the known substance on neurites is highly reproduced. That is, when nerve cells are cultured in the microfluidic device in a state where each of the known substances is individually added to a culture solution, a microscope image of neurites located in the second culture space includes information that a certain effect has been exerted on a site (impaired site) to be affected by each of the known substances.

Therefore, machine learning can be performed by preparing a plurality of teaching data obtained by associating input data for learning, which is data of microscope images themselves of regions (in the second culture spaces) where neurites are present or data obtained by subjecting the microscope images to predetermined image processing, with output data for learning which is information about impaired sites resulting from known substances added to culture spaces of nerve cells corresponding to the microscope images. It should be noted that the known substances may include one that does not impair nerves even when introduced. In this case, teaching data is prepared which is obtained by associating input data for learning, which is data based on a microscope image of neurites cultured in a state where such a known substance is added, with output data for learning which is information that there is no impaired site.

A well-known method can be used for machine learning based on teaching data including the above-described input data for learning and the above-described output data for learning. For example, a learned model is generated by constructing a neural network having an input layer with a plurality of nodes constituted from information about features extracted from image data based on the above-described microscope images, an output layer with a plurality of nodes constituted from information about respective sites belonging to the group of impaired site candidates and information that there is no impaired site, and at least one intermediate layer with a plurality of nodes connecting these input and output layers.

The medicinal agent effect prediction system according to the present invention includes the storage unit that records such a learned model. Therefore, when nerve cells are cultured in such a microfluidic device as described above in a state where a test substance is added, data based on a microscope image of neurites located in the second culture space can be applied to the learned model by inputting the data to the system as input data for assessment. That is, this input data for assessment is the same in quality as the input data for learning used as teaching data for generating a learned model, and therefore a highly reproducible result of assessment can be obtained by applying the input data for assessment to this learned model. In this system, the assessment unit outputs information derived from the probabilities of occurrence of impairments of different sites belonging to the group of impaired site candidates. It should be noted that when the possibility of development of an impairment of nerves resulting from the introduction of this test substance is zero, the above-described probabilities of occurrence of impairments of different sites are all zero.

As described above, as long as nerve cells are cultured in the microfluidic device having the above-described structure in a state where a test substance is added and a microscope image of neurites located in the second culture space separately from the nerve cells is previously obtained, the medicinal agent effect prediction system according to the present invention can automatically calculate information derived from the probabilities of occurrence of impairments of different nerve sites (the values themselves of probabilities of occurrence of impairments or score values based on the values of the probabilities) by arithmetic processing based on data related to this microscope image. This result can be used for assessment of presence or absence of side effects from the early stage of drug discovery, and therefore a safer medicinal agent can be discovered.

The group of impaired site candidates may include axonopathy, neuronopathy, and myelinopathy.

The second culture space of the microfluidic device preferably has a flat shape having a width that allows the neurites growing from the nerve cells to be cultured and a height that prevents the nerve cells from entering thereinto.

The second culture space of the microfluidic device may have a width 10 times or more its height. Preferably, the second culture space has a flat shape having a width seven times or more a diameter of the neurites. Preferably, the second culture space has a height of 5 µm to 80 µm.

The nerve cells may be cells derived from mouse DRG, rat DRG, human ES cells, or human iPS cells.

### EFFECT OF THE INVENTION

The present invention provides a system capable of easily and efficiently predicting the effect of a medicinal agent on nerves.

### BRIEF DESCRIPTION OF THE DRAWINGS

Fig. 1 is a schematic block diagram illustrating a configuration of an embodiment of a medicinal agent effect prediction system according to the present invention.
Fig. 2 is an exploded perspective view of a microfluidic device before manufacture.
Fig. 3 is a plan view of the microfluidic device.
Fig. 4 is a cross-sectional view along line IV-IV of the microfluidic device shown in Fig. 3.
Fig. 5 is a schematic plan view illustrating how nerve cells are cultured in the microfluidic device.
Fig. 6 is a cross-sectional view along line VI-VI of the microfluidic device shown in Fig. 5.
Fig. 7A is a microscope image of neurites at the time when nerve cells are cultured using a culture solution containing Suramin.
Fig. 7B is a microscope image of neurites at the time when nerve cells are cultured using a culture solution containing Oxaliplatin.
Fig. 7C is a microscope image of neurites at the time when nerve cells are cultured using a culture solution containing Vincristine.
Fig. 7D is a microscope image of neurites at the time when nerve cells are cultured using a culture solution containing Sucrose.
Fig. 8 is a schematic diagram illustrating teaching data used for generating a learned model.
Fig. 9 is a conceptual diagram of a learned model generated by a model generation unit.
Fig. 10 is a microscope image of neurites at the time when nerve cells are cultured using a culture solution containing Paclitaxel.
Fig. 11 is a table showing the result of assessment at the time when data derived from the microscope image shown in Fig. 10 is input to the medicinal agent effect prediction system.

### MODE FOR CARRYING OUT THE INVENTION

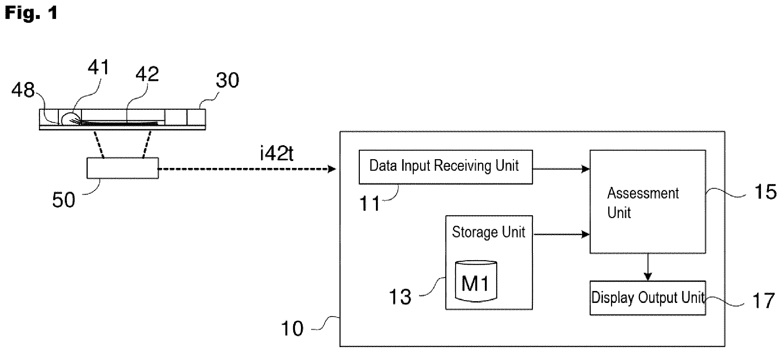

An embodiment of a medicinal agent effect prediction system according to the present invention will be described with reference to the drawings. Fig. 1 is a schematic block diagram illustrating a configuration of the embodiment of the medicinal agent effect prediction system.

As shown in Fig. 1, a medicinal agent effect prediction system 10 includes a data input receiving unit 11, a storage unit 13, an assessment unit 15, and a display output unit 17. The storage unit 13 records a learned model M1. Hereinbelow, the term "medicinal agent effect prediction system 10" is abbreviated as "system 10" as appropriate.

### [Overall configuration of system]

The system 10 has the function of arithmetically calculating the possibility that a test substance, which is not known as to which nerve site is impaired thereby, will impair a nerve site. Hereinbelow, a method for using the system 10 will be described. The following description will be made with reference to peripheral nerves which are a type of nerves, but the same arguments can be made also for central nerves. It should be noted that the term "nerves" herein includes peripheral nerves and central nerves.

As shown in Fig. 1, an observation image of neurites 42 cultured using a culture solution containing a test substance 48 is obtained by a microscope 50, and data based on this observation image (hereinafter referred to as "input data for assessment i42t") is input to the data input receiving unit 11. The data input receiving unit 11 is an interface for inputting image data. If necessary, the data input receiving unit 11 has the function of performing, on inputted image data, processing to convert it into a data format usable in the system 10.

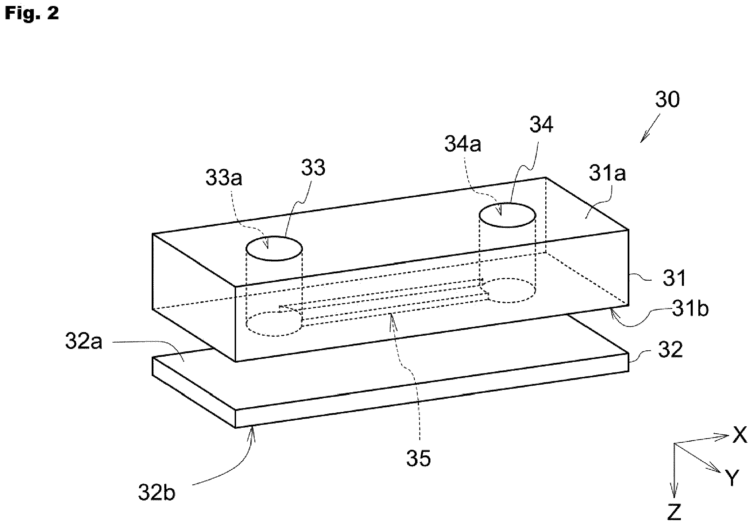

More specifically, nerve cells 41 are cultured using a culture solution containing the test substance 48 in a microfluidic device 30 described later with reference to Fig. 2, and an image of a region including neurites 42 growing from the nerve cells 41 is taken by the microscope 50. The microscope image itself or data obtained by subjecting the microscope image to image processing is input as the input data for assessment i42t to the system 10 through the data input receiving unit 11.

The assessment unit 15 applies this input data for assessment i42t to the learned model M1 recorded in the storage unit 13. The learned model M1 is a model generated by performing machine learning on the basis of a plurality of teaching data obtained by associating previously obtained microscope images of areas where other neurites 42 are present with information about impaired sites shown by the microscope images. That is, the learned model M1 has a neural network constructed therein which receives input data derived from the microscope image of the neurites 42 on its input side and outputs information about an impaired site of the neurites 42 on its output side.

Therefore, by applying the inputted input data for assessment i42t to the learned model M1, it is possible to output stochastically what kind of impairment may be occurring to the neurite 42 from the information appearing in this input data i42t for judgment. The assessment unit 15 outputs the result of the assessment to the display output unit 17, and the result is displayed on the display output unit 17.

The assessment unit 15 is a means for arithmetic processing to perform a calculation using the learned model M1 and output a result thereof by applying the input data for assessment i42t to the learned model M1 and is constituted from, for example, a CPU or an MPU. The storage unit 13 is a memory medium that stores predetermined information including the learned model M1 and is typically a non-volatile memory such as a flash memory or a hard disk. The display output unit 17 is, for example, a monitor.

However, information about the result of assessment by the assessment unit 15 may be sent to a remote location through a communication line such as the Internet not shown.

### [Configuration of microfluidic device]

Next, the configuration of the microfluidic device 30 used for generating the learned model M1 and obtaining the input data for assessment i42t will be described with reference to the drawings. It should be noted that the following drawings merely show schematic illustrations, and the dimensional ratios on the drawings do not necessarily reflect the actual dimensional ratios. Further, the dimensional ratios are not necessarily the same between the drawings.

Fig. 2 is an exploded perspective view of the microfluidic device 30 before completion. The microfluidic device 30 has a first substrate 31 and a second substrate 32 and is manufactured by bonding them together. Fig. 2 corresponds to a perspective view showing both of the substrates (31, 32) just before bonding the second substrate 32 onto the first substrate 31.

The first substrate 31 and the second substrate 32 are formed of a transparent thermoplastic resin. Examples of the thermoplastic resin include polymethyl methacrylate (PMMA), polycarbonate (PC), cycloolefin copolymer (COC), cycloolefin polymer (COP), and polystyrene (PS). Among these, medical-grade COP is particularly preferred. COP is a thermoplastic resin having high transparency, low autofluorescence, and low drug adsorption.

After through-holes (33a, 34a) and a recess 35, which will be described later, are formed by, for example, injection molding, the substrates (31, 32) formed of the above-described material are subjected to an ultraviolet irradiation step and a pressing step to obtain the microfluidic device 30.

The microfluidic device 30 is formed by stacking the first substrate 31 on the second substrate 32 in such a manner that one principal surface 31b of the first substrate 31 partially comes into contact with one principal surface 32a of the second substrate 32 and bonding the first substrate 31 and the second substrate 32 together. The term "principal surface" herein refers to one of surfaces constituting the substrates (31, 32) and having a much larger area than other surfaces. Each of the substrates (31, 32) has two principal surfaces, and these two principal surfaces are opposed to each other.

One of the principal surfaces 31a of the first substrate 31 is located on the opposite side from the second substrate 32 and has ports (33, 34). The other principal surface 31b of the first substrate 31 is in partial contact with the second substrate 32. It should be noted that the recess 35 is formed in the principal surface 31b of the first substrate 31.

In the following description, an XYZ coordinate system is referenced as appropriate in which, in a state where the first substrate 31 and the second substrate 32 are bonded together, a plane parallel to the principal surfaces (31a, 31b) of the first substrate 31 and the principal surfaces (32a, 32b) of the second substrate 32 is defined as an XY plane and a direction orthogonal to the XY plane is defined as a Z direction.

When it is necessary to make a distinction between positive and negative to express a direction herein, the direction is described with a positive or negative sign, such as "+X direction" or "-X direction". When it is not necessary to make a distinction between positive and negative to express a direction, the direction is simply described as "X direction". Namely, when the direction is simply described as "X direction" herein, both "+X direction" and "-X direction" are included. The same applies to a Y direction and a Z direction. It should be noted that the microfluidic device 30 is usually used in such a manner that the Z direction corresponds to a vertical direction, and the -Z direction corresponds to an upward direction.

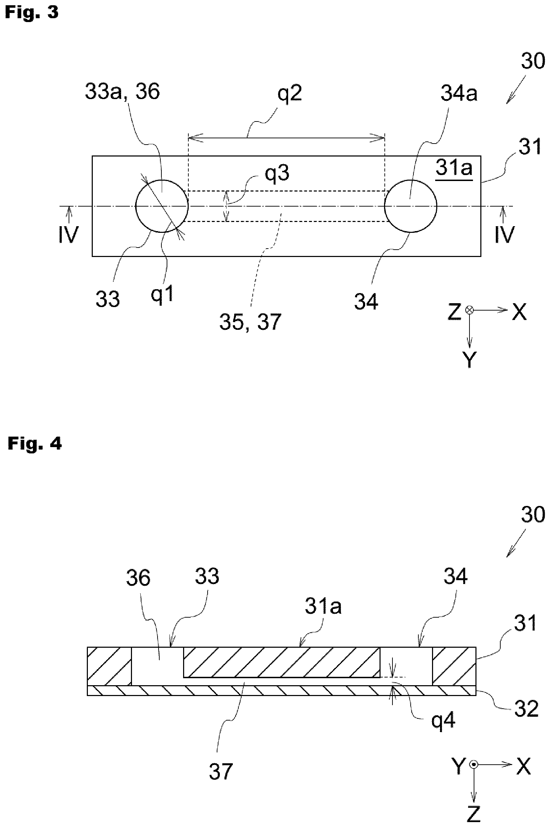

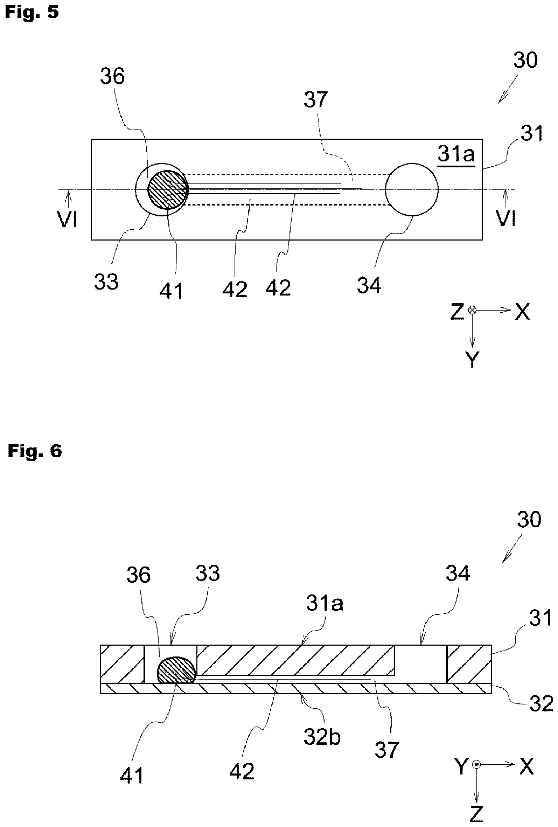

Fig. 3 is a plan view of the microfluidic device 30. Fig. 4 is a cross-sectional schematic view along line IV-IV of the microfluidic device 30 shown in Fig. 3, in which the first substrate 31 is bonded onto the second substrate 32. It should be noted that in the schematic cross-sectional view of Fig. 4, among outlines of both of the substrates (31, 32), only lines appearing in the section are shown to facilitate understanding of the diagram. The same applies to Fig. 6 described later.

Each of the first substrate 31 and the second substrate 32 is a rectangular substrate having principal surfaces that are the same in shape. The principal surfaces of the first substrate 31 and the second substrate 32 have a size of, for example, 10 mm × 20 mm.

The thickness of the first substrate 31 is larger than that of the second substrate 32. The thickness of the first substrate 31 is, for example, 0.2 mm to 10 mm, and is typically 3 mm. The thickness of the second substrate 32 is, for example, 0.1 mm to 2 mm, and is typically 1 mm. It should be noted that the term "thickness" herein corresponds to the length in the Z direction.

As shown in Fig. 2, the through-holes (33a, 34a) are formed in the first substrate 31 so as to extend from the first principal surface 31a toward the second principal surface 31b. In Fig. 2, these through-holes (33a, 34a) having such a shape and disposed so as to be arranged in the X direction are shown by way of example.

The port 33 as an open end of the through-hole 33a and the port 34 as an open end of the through-hole 34a are provided for at least one purpose selected from the purpose of injecting a liquid into the microfluidic device 30 and the purpose of discharging a liquid from the microfluidic device 30. Typically, the port 33 is used as, for example, a liquid inlet, and the port 34 is used as, for example, a liquid outlet.

When the first substrate 31 and the second substrate 32 are bonded together, the through-hole 33a functions as a first culture space 36 (see Fig. 3 and Fig. 4). Here, an example in which the through-hole 33a functions as the first culture space 36 is shown, but the through-hole 34a may function as the first culture space 36. Alternatively, each of the through-hole 33a and the through-hole 34a may function as the first culture space 36.

The through-holes (33a, 34a) are both circular holes extending in the Z direction. A diameter q1 (see Fig. 3) of the through-holes (33a, 34a) is, for example, 0.05 mm to 5 mm, and is typically 2 mm. A separation distance q2 (see Fig. 3) between the through-hole 33a and the through-hole 34a is, for example, 1 mm to 100 mm, and is typically 7 mm.

As described above with reference to Fig. 2, the first substrate 31 has the recess 35 on the second principal surface 31b side. The recess 35 is formed so as to extend in the X direction. The section of the recess 35 perpendicular to its extension direction (X direction) (the section parallel to a YZ plane) has a flat rectangular shape.

The recess 35 communicates with the through-hole 33a and the through-hole 34a. When the first substrate 31 and the second substrate 32 are bonded together, the recess 35 functions as a hollow second culture space 37 sandwiched between both of the substrates (31, 32).

The recess 35 has a slit shape extending in the X direction with a constant width and depth. The recess 35 preferably has a width 10 times or more its depth. That is, the second culture space 37 is formed in such a manner that when it is viewed in its extension direction, a width q3 (see Fig. 3) is preferably 10 times or more a height q4 (see Fig. 4). The second culture space 37 is formed in such a manner that the width q3 is preferably 10 times or more, more preferably 20 times or more the height q4. The width q3 is preferably equal to or less than 100 times the height q4. It should be noted that in the case of a conventional microfluidic device formed of silicone rubber, a flat channel having a width 10 times or more its height is difficult to maintain its height due to the effect of gravity or the like.

As described above, the microfluidic device 30 includes the first culture space 36 and the second culture space 37 provided so as to extend from the first culture space 36. The second culture space 37 is formed in such a manner that when it is viewed in its extension direction, the width q3 is preferably 10 times or more the height q4. Such a configuration makes it possible, when nerve cells 41 are cultured in the first culture space 36, to allow neurites 42 growing from the nerve cells 41 to be located in the second culture space 37 that is different from a space where the nerve cells 41 are cultured (see Fig. 5 and Fig. 6). That is, the microfluidic device 30 makes it possible to culture nerve cells 41 and neurites 42 separately from each other.

Fig. 5 and Fig. 6 are respectively a plan view and a cross-sectional view schematically illustrating how nerve cells 41 are cultured in the microfluidic device 30. Fig. 6 is a cross-sectional view along line VI-VI of the microfluidic device 30 shown in Fig. 5.

Nerve cells 41 are cultured in the first culture space 36, and neurites 42 growing from the nerve cells 41 are cultured in the second culture space 37. Since the microfluidic device 30 has such a structure as described above, the second culture space 37 is capable of culturing the neurites 42 while preventing the nerve cells 41 themselves from entering thereinto.

The nerve cells 41 are classified by the presence or absence of myelin sheath, diameter, conduction velocity, and the like. In the case of type A nerve fibers having a myelin sheath, type Aα nerve fibers have a diameter of 13 µm to 22 µm, type Aβ nerve fibers have a diameter of 8 µm to 13 µm, type Aγ nerve fibers have a diameter of 4 µm to 8 µm, and type Aδ nerve fibers have a diameter of 1 µm to 4 µm. Automatic nerve fibers having a myelin sheath are called type B nerve fibers and have a diameter of 1 µm to 3 µm. Type C nerve fibers (C fibers) having no myelin sheath have a diameter of 0.2 µm to 1.0 µm.

Since the second culture space 37 is formed to have a flat sectional shape, as shown in Fig. 5, the neurites 42 do not form a bundle-like structure but are cultured so that the respective neurites 42 are arranged in a width direction (Y direction).

When constituted only from axons (when having no myelin), the neurites 42 have a diameter of 0.2 µm to 1.5 µm. When constituted from axons covered with myelin, the neurites 42 have a diameter of 1 µm to 22 µm. When the width q3 (see Fig. 3) of the second culture space 37 is seven times or more than the diameter of the neurites 42, the neurites 42 can grow without coming into contact with the side walls of the second culture space 37.

The width q3 (see Fig. 3) of the second culture space 37 is preferably 50 µm to 8000 µm, more preferably 500 µm to 1000 µm. The width q3 of the second culture space 37 is typically 800 µm.

The height q4 (see Fig. 4) of the second culture space 37 is preferably 5 µm to 80 µm, more preferably 20 µm to 80 µm. The height q4 of the second culture space 37 is typically 40 µm.

### [Learned model]

Next, a method for preparing the learned model M1 will be described.

When a specific impairment occurs in peripheral nerves, the shape and structure of neurites 42 are changed due to the impairment. Some substances to be contained as medicinal agents are known to cause what kind of impairments to peripheral nerves they do or do not cause (hereinafter referred to as "known substances"). As described above, the microfluidic device 30 is capable of culturing nerve cells 41 and neurites 42 separately from each other. That is, when nerve cells 41 are cultured in the microfluidic device 30, more specifically in the first culture space 36 in a state where a known substance is introduced thereinto and neurites 42 growing from the nerve cells 41 and located in the second culture space 37 are observed, the neurites 42 affected by the known substance can be observed.

It should be noted that from the viewpoint of facilitating observation with a microscope, staining may be performed using a predetermined chemical solution before observation. An example of the method of such staining is fluorescent antibody staining targeting β-tubulin III as a biomarker of nerve axons and Myelin Basic Protein (MBP) as a biomarker of myelin.

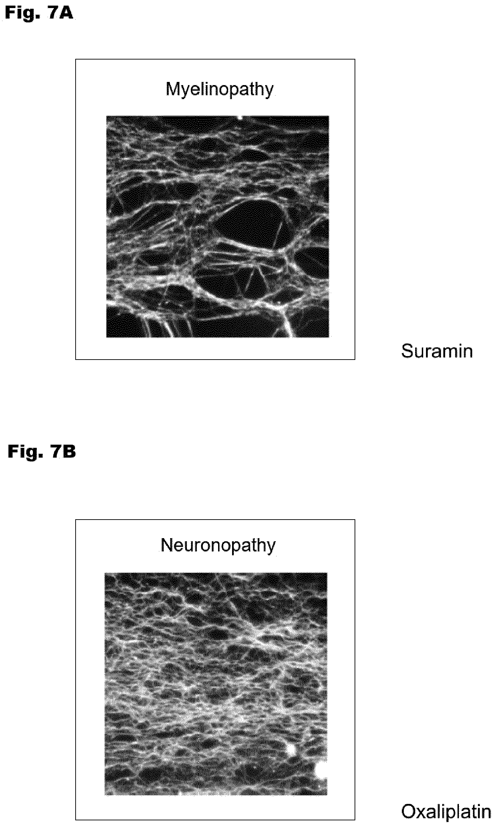

Fig. 7A to Fig. 7D are observation images obtained by culturing nerve cells 41 in the microfluidic devices 30 using culture solutions respectively containing different known substances and observing, with a microscope, neurites 42 located in the second culture spaces 37. It should be noted that rat DRG cells are herein used as nerve cells 41, but various cells such as mouse DRG cells, human ES cells, or human iPS cells can be used.

Fig. 7A is a microscope image of neurites 42 at the time when nerve cells 41 are cultured using a culture solution containing Suramin as a known substance. The time of exposure was 24 hours. Suramin is known to cause myelinopathy (myelin disorder).

Fig. 7B is a microscope image of neurites 42 at the time when nerve cells 41 are cultured using a culture solution containing Oxaliplatin as a known substance. The time of exposure was 24 hours. Oxaliplatin is known to cause neuronopathy.

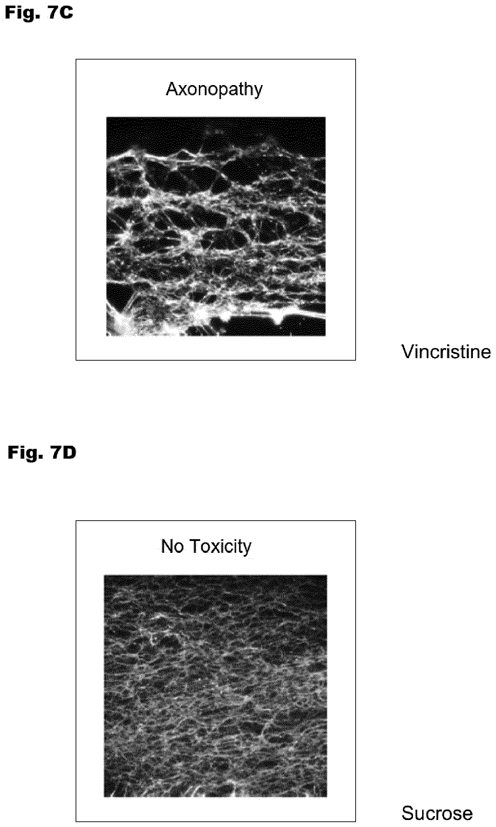

Fig. 7C is a microscope image of neurites 42 at the time when nerve cells 41 are cultured using a culture solution containing Vincristine as a known substance. The time of exposure was 24 hours. Vincristine is known to cause axonopathy.

Fig. 7D is a microscope image of neurites 42 at the time when nerve cells 41 are cultured using a culture solution containing Sucrose as a known substance. Sucrose is known not to be toxic to peripheral nerves.

As described above, a plurality of microscope images of neurites 42 located in the second culture spaces 37 can be obtained by culturing nerve cells 41 in the microfluidic devices 30 using culture solutions respectively containing different known substances. It should be noted that from the viewpoint of increasing learning accuracy, a plurality of microscope images of neurites 42 cultured using culture solutions containing the same known substance may be obtained.

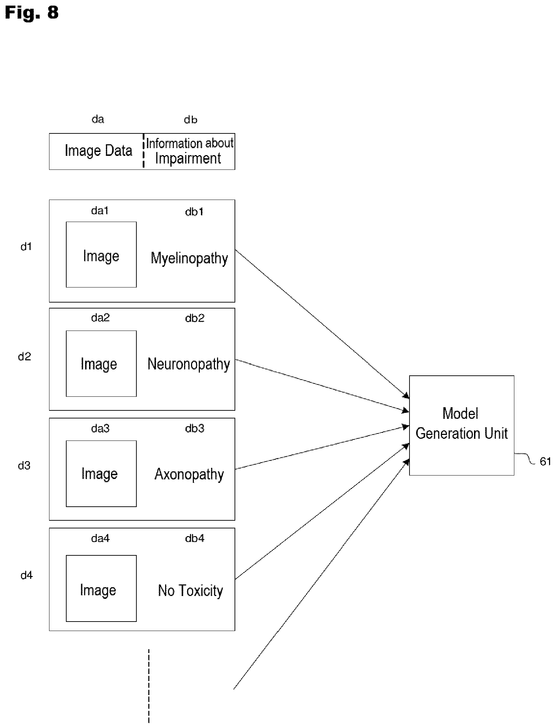

The above-described method makes it possible to obtain a plurality of data columns that are combinations of image data da derived from a microscope image of neurites 42 cultured using a culture solution containing a known substance and information db about an impairment of peripheral nerves resulting from the known substance (information about an impaired site or information that there is no impaired site) which corresponds to the image data da. As shown in Fig. 8, the data columns are introduced into a model generation unit 61 to perform machine learning. The model generation unit 61 is an arithmetic processing means to perform machine learning by arithmetic processing, and is, for example, a CPU or an MPU. It should be noted that the model generation unit 61 may be included in the medicinal agent effect prediction system 10 itself or may be included in another system.

The model generation unit 61 performs machine learning on the basis of a plurality of teaching data including, as input data for learning, the image data da based on microscope images and, as output data for learning, the data db associated with the input data for learning and including information about impairments of peripheral nerves. It should be noted that the term "machine learning" herein refers to learning performed only by a machine (especially, a computer) without the intervention of human thought.

As a typical method of learning used for machine learning, a neural network can be used. From the viewpoint of increasing the accuracy of assessment, a layered neural network is preferably used which has at least one intermediate layer between an input layer and an output layer. However, another learning method may be used. Examples of a usable learning method other than a neural network include linear regression, decision tree, support vector regression, and an ensemble method. These may be used singly or in combination of two or more of them.

The machine learning can be performed using, for example, TensorFlow (trademark), Keras (trademark), Pytorch, or Matlab (trademark).

From the viewpoint of increasing the accuracy of assessment, the number of samples of the teaching data is preferably 10,000 or more, more preferably 100,000 or more, particularly preferably 1,000,000 or more. From the viewpoint of increasing the accuracy of assessment, the number of times of learning is preferably 100 or more, more preferably 500 or more, particularly preferably 1,000 or more.

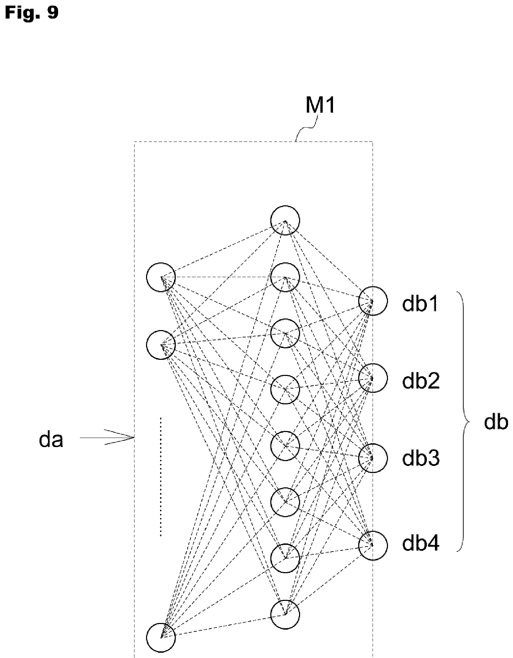

Fig. 9 is a conceptual diagram of the learned model M1 generated by the model generation unit 61. A network is constructed by using features based on the image data da based on microscope images of neurites 42 as an input layer, the data db relating to impairments of the neurites 42 as an output layer, and an intermediate layer connecting them. It should be noted that as the data db relating to impairments, four nodes are set which are myelinopathy db1, neuronopathy db2, axonopathy db3, and no toxicity db4. That is, in this embodiment, three kinds of sites, myelin sheath, cell body, and axon are expected as impaired site candidates. It should be noted that the features are extracted from the image data da by using a versatile method performed in machine learning based on image data.

Axonopathy db1 is a phenomenon in which axons themselves are impaired. Neuronopathy db2 is a phenomenon in which cell bodies are impaired and the effect thereof spreads through axons so that nerve cells are entirely impaired. Myelinopathy (myelin disorder) db3 is a phenomenon in which myelin sheaths covering and protecting axons are impaired. That is, effects on the linear shape, thickness, and branch shape of the neurites 42, the distance between the neurites 42, the distance between the branches of the neurites 42, and the uniformity, dispersibility, collectivity of the network of nerves are different depending on the type of impairment. Such effects appear in the image data da derived from microscope images of the neurites 42. That is, by extracting features from the image data da, the learned model M1 based on the relationship between the impaired sites and the features is generated.

The thus constructed learned model M1 is stored in the storage unit 13 of the system 10.

It should be noted that in the above embodiment, the learned model M1 includes an output layer having four nodes including three kinds of impaired sites of peripheral nerves and a case where no impairment occurs, but the number of kinds of impaired sites can be 4 or more.

### [Verification]

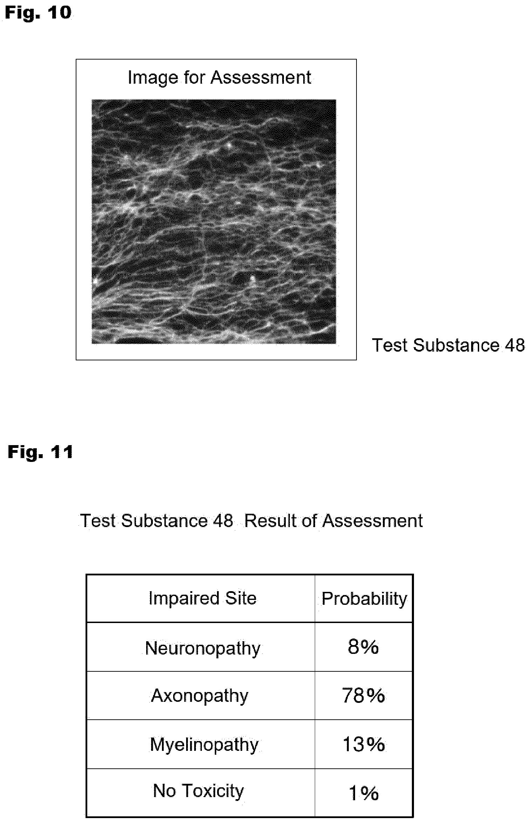

Paclitaxel known to cause axonopathy of peripheral nerves was used as an example of the test substance 48, and nerve cells 41 were cultured in the microfluidic device 30 using a culture solution containing the test substance 48. Then, neurites 42 growing from the nerve cells 41 and located in the second culture space 37 were observed by the microscope 50 to obtain an observation image. The observation image is shown in Fig. 10.

Image data based on the observation image was input as input data for assessment i42t to the system 10 through the data input receiving unit 11. In the system 10, the assessment unit 15 applied the input data for assessment i42t to the learned model M1. That is, features based on the input data for assessment i42t were applied to the input layer of the learned model M1. As a result, the result of assessment shown in Fig. 11 was output from the display output unit 17. From this, it can be confirmed that the test substance 48 has a high possibility of causing axonopathy. This result of assessment was correct in light of the fact that the test substance 48 is Paclitaxel, which is known to cause axonopathy.

When an assessment test was performed 5776 times by the medicinal agent effect prediction system 10 using Paclitaxel images as input data for assessment i42t, the system 10 reached a correct answer (axonopathy) 1226 times (21.2%). However, among 2027 assessment tests in which Paclitaxel was not assessed to have no toxicity, the probability of being assessed as axonopathy was 60.5% (1226/2027). The percentage of correct answers is further improved by increasing the number of samples of teaching data and the number of times of learning.

### DESCRIPTION OF REFERENCE SIGNS

- 10: Effect prediction system
- 11: Data input receiving unit
- 13: Storage unit
- 15: Assessment unit
- 17: Display output unit
- 30: Microfluidic device
- 31: First substrate
- 31a: First principal surface of first substrate
- 31b: Second principal surface of first substrate
- 32: Second substrate
- 32a: Principal surface of second substrate
- 33: Port
- 33a: Through-hole
- 34: Port
- 34a: Through-hole
- 35: Recess
- 36: First culture space
- 37: Second culture space
- 41: Nerve cell
- 42: Neurite
- 48: Test substance
- 50: Microscope
- 61: Model generation unit

## Claims

1. A medicinal agent effect prediction system comprising:
a microfluidic device having a first culture space capable of culturing nerve cells and a second culture space that is provided so as to extend from the first culture space and that is capable of culturing, separately from the nerve cells, neurites growing from the nerve cells;
a storage unit that records a learned model generated by machine learning based on a plurality of teaching data obtained by associating input data for learning with output data for learning: the input data for learning being data which is based on microscope images of the neurites located in the second culture spaces and respectively growing from the nerve cells cultured in the first culture spaces of a plurality of the microfluidic device prepared for learning in a state where each of known substances that impair known nerve sites belonging to a group of impaired site candidates or do not impair any nerve site is individually added to a culture solution, the output data for learning being data which is information about impaired nerve sites resulting from the known substances added to the first culture spaces in which the nerve cells corresponding to the microscope images are cultured or information that there is no impaired site;
a data input receiving unit that receives input of input data for assessment based on a microscope image of the neurites located in the second culture space and growing from the nerve cells cultured in the first culture space of the microfluidic device prepared for assessment in a state where a test substance, which is not known as to which nerve site is impaired thereby, is added to a culture solution; and
an assessment unit that outputs information derived from probability of occurrence of impairment of each nerve site belonging to the group of impaired site candidates resulting from introduction of the test substance, by applying the input data for assessment to the learned model.

2. The medicinal agent effect prediction system according to claim 1, wherein the group of impaired site candidates include axonopathy, neuronopathy, and myelinopathy.

3. The medicinal agent effect prediction system according to claim 1 or 2, wherein the second culture space of the microfluidic device has a flat shape having a width that allows the neurites growing from the nerve cells to be cultured and a height that prevents the nerve cells from entering thereinto.

4. The medicinal agent effect prediction system according to claim 1 or 2, wherein the second culture space of the microfluidic device has a flat shape having a width seven times or more a diameter of the neurites growing from the nerve cells.

5. The medicinal agent effect prediction system according to claim 1 or 2, wherein the second culture space of the microfluidic device has a width 10 times or more its height.

6. The medicinal agent effect prediction system according to claim 1 or 2, wherein the nerve cells are cells derived from mouse DRG, rat DRG, human ES cells, or human iPS cells.
